(19) Europäisches Patentamt
European Patent Office
Office européen des brevets

(11) EP 2 979 692 A1

(12) EUROPEAN PATENT APPLICATION

(43) Date of publication:
**03.02.2016 Bulletin 2016/05**

(51) Int Cl.:
*A61K 9/20* (2006.01) *A61K 31/52* (2006.01)

(21) Application number: **14179182.2**

(22) Date of filing: **30.07.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **SANDOZ AG**
**4056 Basel (CH)**

(72) Inventor: **The designation of the inventor has not yet been filed**

(74) Representative: **Englmeier, Ludwig**
**c/o Sandoz International GmbH**
**Global Patents Department**
**Industriestrasse 25**
**83607 Holzkirchen (DE)**

(54) **Dosage form comprising a pressure sensitive solid form of an active pharmaceutical ingredient**

(57) The present invention concerns a dosage form comprising a pressure sensitive solid form of an active pharmaceutical ingredient, a first disintegrant having elastic properties, a second disintegrant and optionally a pharmaceutical excipient.

**FIG. 1**

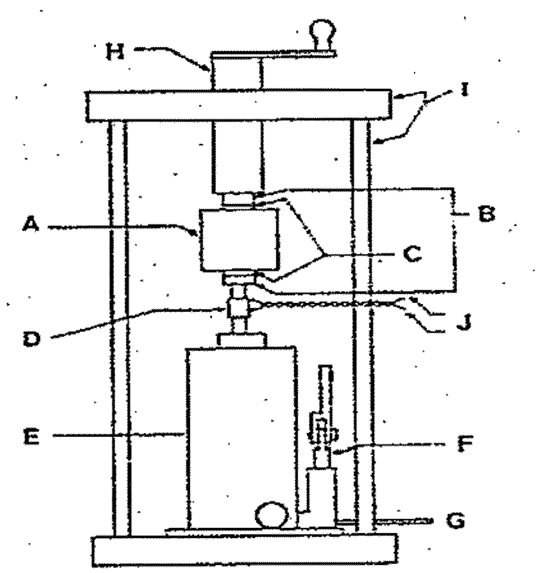


Schematic of apparatus for tensile strength determination by transverse compression.

EP 2 979 692 A1

## Description

The present invention concerns a compressed solid dosage form comprising a pressure sensitive solid form of an active pharmaceutical ingredient, a first disintegrant having elastic properties, a second disintegrant and optionally a further pharmaceutical excipient.

## Background of the Invention

An important property of a medicament is the bioavailability of the active pharmaceutical ingredient contained within the medicament. Bioavailability refers to the extent and rate at which the active moiety (drug or metabolite) enters systemic circulation, thereby accessing the site of action. The bioavailability of a medicament is of great importance in view of the specific medical indication for which it has been developed.

Various physiological factors reduce the availability of medicaments prior to their entry into the systemic circulation, such disease states affecting liver metabolism or gastrointestinal function. Of course, also the formulation of the medicament has a great influence on the bioavailability.

One important factor influencing the bioavailability of a medicament is the solubility of an active pharmaceutical ingredient. Changes in solubility of an active pharmaceutical ingredient (API) can have a great effect on the absorption and consequently on the bioavailability of the active pharmaceutical ingredient, in particular in those cases where the active pharmaceutical ingredient has a low solubility. The bioavailability of active pharmaceutical ingredients having low solubility is limited by their dissolution rate. Hence, changes in their dissolution rate will also have an impact on their bioavailability.

There are many factors that influence the solubility and/or dissolution rate of an active pharmaceutical ingredient, inter alia the formulation process, particle size, the crystal form and the degree of crystallinity.

Generally, amorphous forms of an active pharmaceutical ingredient have a higher solubility than crystalline forms of the same active pharmaceutical ingredient. However, also different crystalline forms (polymorphs) of the same active pharmaceutical ingredient can show a different solubility and/or dissolution rate. Hence, different polymorphs may lead to a different bioavailability and, thus, may even be indicated for treating different medical conditions.

It is known that specific solid state forms of some active pharmaceutical ingredients can be at least partially transformed into other forms of the same active pharmaceutical ingredient by applying pressure on the active pharmaceutical ingredient. This effect can be problematic during the preparation of compressed solid dosage forms, when quite high pressures are generally applied. In such a situation pressure sensitivity of a particular solid state form of an active pharmaceutical ingredient typically discourages the use of such a solid state form for e.g. tablet preparation, even though the pressure sensitive solid state form might otherwise have very desirable properties for pharmaceutical formulation.

For example, WO 2013/134288 describes the at least partial conversion of polymorphic Form I of idelalisib to polymorphic Form II upon compression, making form I of idelalisib an unlikely choice for the development of a tablet formulation.

It is therefore highly desirable to provide a process that would allow production of a compressed dosage form from a pressure sensitive API in such a way that during the compression process the pressure sensitive API does not change its degree of structural order. This is because a change could negatively influence the bioavailability or other desired properties of the solid state form in a compressed dosage form.

It is known that different solid forms of an active pharmaceutical ingredient can have different characteristics not only in view of solubility and/or dissolution. For example, an at least partial change of the degree of structural order of a solid form of the active pharmaceutical ingredient during the production of a compressed solid dosage form can have a negative impact on the storage stability and/or photostability of the active pharmaceutical ingredient after compression and consequently also of the resulting compressed dosage form. Furthermore, an at least partial change of the degree of structural order of a pressure sensitive solid form of the active pharmaceutical ingredient during the production of a compressed solid dosage form can also have negative implications for relevant properties of the active pharmaceutical ingredient, such as compactability or compatibility with other excipients, thus impeding the process for the preparation of the compressed solid dosage form. In summary, there are various reasons why enabling the use of pressure sensitive solid forms of an API for the preparation of compressed pharmaceutical dosage forms, while preserving the particular pressure sensitive solid form of the API during the compression process, would greatly benefit the pharmaceutical practitioner.

It is thus an object of the present invention to provide a compressed solid dosage form comprising a pressure sensitive solid form of an active pharmaceutical ingredient in which the pressure sensitive solid form of the active pharmaceutical ingredient is preserved during the production of the compressed solid dosage form.

According to the present invention, the above problem is unexpectedly overcome by a compressed solid dosage form comprising a pressure sensitive solid form of an active pharmaceutical ingredient, a first disintegrant having elastic properties and a second disintegrant. Further, the above problem can be overcome by granules obtained by a process

comprising the steps described in the present invention. Finally, the above drawback can be overcome by a specific process for the production of a compressed solid dosage form comprising a pressure sensitive solid form of an active pharmaceutical ingredient.

Thus, the subject of the present invention is a compressed solid dosage form comprising:

a) a pressure sensitive solid form of an active pharmaceutical ingredient,
b) a first disintegrant having elastic properties,
c) a second disintegrant,
d) optionally a further pharmaceutical excipient, and
e) optionally a coating,

wherein the weight ratio of the pressure sensitive solid form of an active pharmaceutical ingredient (a) to the first disintegrant having elastic properties (b) is from 10:1 to 1:7 and wherein a pressure sensitive solid form is defined as a solid form which modifies its degree of structural order under a pressure of 30 MPa.

Further, the present invention relates to a process for the production of a compressed solid dosage form comprising a pressure sensitive solid form of an active pharmaceutical ingredient comprising the steps of

I) providing a pressure sensitive solid form of an active pharmaceutical ingredient a), a first disintegrant b) having elastic properties and optionally a further pharmaceutical excipient d),
II) mixing and preferably granulating the mixture of step I), preferably with a granulation liquid, and
III) optionally drying the granules from step II),

wherein a pressure sensitive solid form is defined as a form which modifies its degree of structural order under a pressure of 30 MPa.

A further subject of the present invention is granules obtainable by a process comprising the steps of

j) providing a pressure sensitive solid form of an active pharmaceutical ingredient a), a first disintegrant b) having elastic properties and optionally a further pharmaceutical excipient,
jj) mixing and granulating the mixture from step j) with a granulation liquid,
jjj) drying the granules from step jj),

wherein a pressure sensitive solid form is defined as a form which modifies its degree of structural order under a pressure of 30 MPa.

Description of the Figure

FIG. 1 shows a schematic of apparatus for tensile strength determination by transverse compression. A, test specimen, i.e. square tablet; B, platens, 0.4 width of tablet; C, pads on platens; D, load cell; E, hydraulic jack; F, hydraulic piston for hand pumping; G, line to power-driven hydraulic pump (constant flow rate); H, screw, height adjustment for top platen; I, frame of hydraulic jack; J, leads for strain gauges of load cell.

**Detailed Description of the Invention**

For the avoidance of doubt, it is hereby stated that the information disclosed earlier in this specification under the heading "Background" is relevant to the invention and is to be read as part of the disclosure of the invention.

Throughout the description and claims of this specification, the singular encompasses the plural unless the context otherwise requires. In particular, where the indefinite article is used, the specification (which term encompasses both the description and the claims) is to be understood as contemplating plurality as well as singularity, unless the context requires otherwise.

All of the features disclosed in this specification (including any accompanying claims, abstract and drawings) and/or all of the steps of any process so disclosed may be combined in any combination, except combinations where at least some of such features and/or steps are mutually exclusive. The invention is not restricted to the details of any foregoing embodiments. The invention extends to any novel feature, or any novel combination of the features disclosed in this specification (including any accompanying claims, abstract and drawings), or to any novel step, or any novel combination of the steps of any process so disclosed.

In the context of the present invention, the term "active pharmaceutical ingredient" (API) is defined as a substance

used in a finished pharmaceutical dosage form (medicament) intended to furnish pharmacological activity or to otherwise have a direct effect on the diagnosis, cure, mitigation, treatment or prevention of disease, or to have direct effect in restoring, correcting or modifying physiological functions in human beings. As used herein, the active pharmaceutical ingredient can refer to its pharmaceutically acceptable salts, hydrates or solvates thereof as well as the free base/acid thereof.

In the context of the present invention, the term "a pressure sensitive solid form" is defined as an amorphous or crystalline form of an active pharmaceutical ingredient which modifies its degree of structural order (change of atomic structure) under a pressure of 30 MPa (Mega Pascal). This definition does not exclude pressure sensitive solid forms which change their structural order already when a lower pressure than 30 MPa is applied but also change their structural order under a pressure of 30 MPa according to the test conditions below. In order to test whether a form of an active pharmaceutical ingredient is pressure sensitive, the following procedure is applied. A tablet hydraulic press is used. Preferably, the test is carried out with 500 mg pure form of an active pharmaceutical ingredient. The pressure is applied for 10 minutes at 25 °C and 50 % humidity. The effect of compression is evaluated by x-ray powder diffraction (XRPD), measured as described below. This means that an x-ray powder diffractogram of the solid form of the active pharmaceutical ingredient before compression is compared to an x-ray powder diffractogram of the active pharmaceutical ingredient taken directly after compression. If the two diffractograms are identical within statistical error and/or if differences due to changes in sample orientation are appropriately considered, the solid form is regarded as being not pressure sensitive.

Additionally, a differential scanning calorimetry (DSC) thermogram is measured before the compression and one DSC thermogram of the solid form of the API is measured directly after compression. DSC thermograms can be preferably obtained using a DSC Q100 V8.2 Build 268 (TA Instruments, USA) under a nitrogen gas flow of 50 mL/min. Calibration of the DSC instrument can be carried out using indium (for temperature and enthalpy) and sapphire (for heat capacity) as standards. Sample powder of a pure solid form of an API (5 mg, with an accuracy of 0.05 mg) is crimped in aluminium pans with sealed lids at a humidity of 50% and heated at a scanning rate of 20°C/min from 25°C to 300°C. The heat capacity change at the glass transition temperature Tg (midpoint) can be determined using a TA Universal Analysis 2000 software. If the two thermograms are identical within statistical error, the solid form is regarded as being not pressure sensitive.

DSC thermograms are especially suited for measuring an at least partial conversion from a crystalline form of an API to an amorphous form, since already small conversions to an amorphous form can be detected in form of a glass transition temperature which is typical for amorphous forms. Additionally, an at least partial conversion of a specific polymorph to another polymorph can also be detected by DSC in form of different melting points corresponding to the different polymorphs. Similarly, an at least partial conversion of an amorphous from of an API to a crystalline form of the API can be detected by the presence beside of the glass transition temperature of a defined melting point.

If differences can be observed reproducibly by the skilled person, that is if a change in the atomic structure of at least a detectable subset of the tested sample is causative for the observed differences, for example in a small but detectable transformation from pure amorphous API to an API which is at least partly crystalline or from a crystalline API to an API further containing detectable amounts of amorphous API or from an API consisting of a particular polymorph to an API further containing at least detectable amounts of another polymorph, then the solid form is regarded as being pressure sensitive.

A solid form of an API is regarded as pressure sensitive when at least one, and preferably both, of the two methods (XRPD and/or DSC) provides a positive result in view of an at least partial transformation under the assay conditions as disclosed above.

For a transformation from an API consisting of a single polymorph to an API further containing at least detectable amounts of another polymorph of said API the appearance of new peaks in the XRPD which are characteristic for the new polymorph under the given assay conditions is indicative of pressure sensitivity of the particular polymorph of said API. Alternatively or preferably additionally, for a transformation from an API consisting of a single polymorph to an API further containing at least detectable amounts of another polymorph of said API the appearance of a new melting point in the DSC thermogram which is characteristic for the new polymorph under the given assay conditions is indicative of pressure sensitivity of the particular polymorph of said API.

For a transformation from an API consisting of a particular polymorph to an API further containing at least detectable amounts of the amorphous form of said API the decrease of the signal to background ratio of the peaks in the XRPD which are characteristic for the particular polymorph by a factor of 2 (measured at the middle of the peak width at the basis of the peak) and/or even the disappearance of at least two peaks in the XRPD which are characteristic for the particular polymorph under the given assay conditions is indicative of pressure sensitivity of the particular polymorph of said API. Alternatively or preferably additionally, for a transformation from an API consisting of a particular polymorph to an API further containing at least detectable amounts of the amorphous form of said API the detection of a glass transition temperature which is characteristic for the amorphous form under the given assay conditions by DSC is indicative of pressure sensitivity of the particular polymorph of said API.

For a transformation from an API consisting of pure amorphous API to an API further containing at least detectable amounts of a crystalline form of said API the appearance of peaks in the XRPD which are characteristic for a crystalline form of said API under the given assay conditions is indicative of pressure sensitivity of the pure amorphous form of said API. Alternatively or preferably additionally, for a transformation from an API consisting of pure amorphous API to an API further containing at least detectable amounts of a crystalline form of said API the appearance of a clearly defined melting point which is characteristic for a crystalline form of said API under the given assay conditions by DSC is indicative of pressure sensitivity of the pure amorphous form of said API.

Preferably, the XRPD pattern can be obtained with a PANalytical X'Pert MPD PRO diffractometer equipped with a $\theta/\theta$ coupled goniometer in transmission geometry, Cu- $K\alpha 1/K\alpha 2$ radiation (wavelength 0.15419 nm) with a focusing mirror, a solid state PIXcel detector and reflectance stage with spinning. The pattern can be recorded at a tube voltage of 45 kV and a tube current of 40 mA, applying a step size of 0.013° $2\theta$ with 80s per step (255 channels) in the angular range of 2° to 40° $2\theta$ at ambient conditions (25°C). A typical precision of the $2\theta$ values is in the range of about $\pm$ 0.2° $2\theta$. Thus a diffraction peak that appears at 5.0° $2\theta$ can appear between 4.8 and 5.2° $2\theta$ on most X-ray diffractometers under standard conditions. Samples can be prepared for analysis by distributing solid material as a thin layer on a silicon holder. Each holder can be then mounted on a reflectance/transmittance stage and rotated during data acquisition. It should be understood that relative intensities can vary depending on a number of factors, including sample preparation, mounting and the instrument and analytical procedure and settings used to obtain the spectrum.

"Crystalline" as used herein defines a solid material whose constituent atoms, molecules, or ions are arranged in an ordered pattern extending in all three spatial dimensions and thus exhibit a periodic arrangement over a great range.

The pressure sensitive solid form of an active pharmaceutical ingredient in the dosage form of the invention may preferably consist of a crystalline pressure sensitive solid form of an active pharmaceutical ingredient. Alternatively, the active pharmaceutical ingredient may also contain small amounts of non-crystalline (= amorphous) components - which are not pressure sensitive -, provided that a defined melting point of the pressure sensitive solid form of the active pharmaceutical ingredient can be detected in a DSC.

The pressure sensitive solid form of an active pharmaceutical ingredient in the dosage form of the invention may preferably consist of a non-crystalline pressure sensitive solid form of an active pharmaceutical ingredient. Alternatively, the active pharmaceutical ingredient may also contain small amounts of crystalline components which are not pressure sensitive.

"Polymorphism" as used herein is the ability of a solid material to exist in more than one form or crystal structure. Different polymorphs of the same API typically exhibit different melting points, solubilities, X-ray crystal and diffraction patterns.

The pressure sensitive solid form of an active pharmaceutical ingredient in the dosage form of the invention may consist of a pressure sensitive polymorph of an active pharmaceutical ingredient.

Thus, in a preferred embodiment, the pressure sensitive solid form of an active pharmaceutical ingredient a) is a polymorphic form which converts partially into another polymorphic form upon pressure, i.e. under the conditions described above. In a further embodiment, the pressure sensitive solid form of an active pharmaceutical ingredient a) is a polymorphic form which converts completely into another polymorphic form upon pressure.

In a further preferred embodiment, the pressure sensitive solid form of an active pharmaceutical ingredient a) is an amorphous form which converts partially into a crystalline form upon pressure, i.e. under the conditions described above. In a further embodiment, the pressure sensitive solid form of an active pharmaceutical ingredient a) is an amorphous form which converts completely into a crystalline form upon pressure.

In yet a further preferred embodiment, the pressure sensitive solid form of an active pharmaceutical ingredient a) is a crystalline form which converts partially into an amorphous form upon pressure, i.e. under the conditions described above. In yet a further preferred embodiment, the pressure sensitive solid form of an active pharmaceutical ingredient a) is a crystalline form which converts completely into an amorphous form upon pressure.

As used herein, a partial conversion means that the pressure sensitive solid form is converted under the conditions described above to a detectable extent.

As used herein, a complete conversion means that the pressure sensitive solid form is converted under the conditions described above to an extent that the starting pressure sensitive solid form is no longer detectable by XRPD.

In a particularly preferred embodiment, the dosage form of the invention comprises a pressure sensitive solid form of an active pharmaceutical ingredient as the sole active pharmaceutical ingredient.

In a further particularly preferred embodiment, the dosage form of the invention comprises a pressure sensitive solid form of an active pharmaceutical ingredient as the sole active pharmaceutical ingredient and as the sole solid form of the active pharmaceutical ingredient.

In another preferred embodiment, the dosage form of the invention can comprise a pressure sensitive solid form of an active pharmaceutical ingredient in combination with further active pharmaceutical ingredient(s).

As described above, an at least partial transformation of a pressure sensitive solid form into a more stable form of an active pharmaceutical ingredient can have an influence on the solubility, and thus on the bioavailability of the active

pharmaceutical ingredient. Variations of solubility are especially relevant for active pharmaceutical ingredients with low solubility and high or low permeability. A generally used system for classifying active pharmaceutical ingredients on the basis of their solubility and permeability is the Biopharmaceutics Classification System (BCS).

The BCS is a guide for predicting the intestinal drug absorption provided by the U.S. Food and Drug Administration. This system restricts the prediction using the parameters solubility and intestinal permeability. The solubility classification is based on a United States Pharmacopoeia (USP) aperture. The intestinal permeability classification is based on a comparison to the intravenous injection.

According to the Biopharmaceutics Classification System, drug substances are classified as follows:

- Class I - high permeability, high solubility
    - Those compounds are well absorbed and their absorption rate is usually higher than excretion.
- Class II - high permeability, low solubility
    - The bioavailability of those products is limited by their dissolution rate. A correlation between the in vivo bioavailability and the in vitro dissolution can be found.
- Class III - low permeability, high solubility
    - The absorption is limited by the permeation rate but the drug is dissolved very fast. If the formulation does not change the permeability or gastro-intestinal duration time, then class I criteria can be applied.
- Class IV - low permeability, low solubility
    - Those compounds have a poor bioavailability. Usually they are not well absorbed over the intestinal mucosa and a high variability is expected.

Preferably, the active pharmaceutical ingredient a) comprised in the compressed solid dosage form of the present invention is selected from the BCS class II or IV. As explained above, the bioavailability of active pharmaceutical ingredients selected from BCS class II and IV is particularly affected by the solubility and/or dissolution characteristics of the solid form of the API.

Thus, it is highly beneficial that the present invention provides the possibility to use a pressure sensitive solid form of an API, which pressure sensitive solid form has a higher solubility than non-pressure sensitive solid forms of said API, for the preparation of a compressed dosage form, in particular for an active pharmaceutical ingredient selected from BCS class II and IV.

It is also highly beneficial that the present invention provides the possibility to use a pressure sensitive solid form of an API, which pressure sensitive solid form dissolves faster than non-pressure sensitive solid forms of said API, for the preparation of a compressed dosage form, in particular for an active pharmaceutical ingredient selected from BCS class II and IV.

A further advantage of the compressed dosage form of the invention in particular for active pharmaceutical ingredients selected from BCS class II and IV is provided by the geometrical distribution of the two disintegrants, assuring fast release of the pressure sensitive solid form of the API from the compressed dosage form.

Preferably, the active pharmaceutical ingredient a) is selected from caffeine, indomethacin, sulfobenzamid, paroxetine, theophylline, amoxicillin, cephalexin, cefixime, piroxicam, acetaminophen, chlorpropamid, maprotiline and idelalisib. Alternatively, the active pharmaceutical ingredient a) is selected from caffeine, indomethacin, sulfobenzamid, paroxetine, theophylline, amoxicillin, cephalexin, cefixime, piroxicam, acetaminophen, chlorpropamid and maprotiline. More preferably, the active pharmaceutical ingredient a) is selected from caffeine, sulfobenzamid, paroxetine, amoxicillin, cephalexin, cefixime, piroxicam, acetaminophen, chlorpropamid and maprotiline. In a preferred embodiment, the active pharmaceutical ingredient is idelalisib.

In an embodiment of the invention, the compressed solid dosage form comprises idelalisib, which is to be used in combination with a rituximab infusion and/or a bendamustine infusion.

In a further embodiment, the active pharmaceutical ingredient a) does not comprise indomethacin and/or theophylline.

In a further embodiment, the active pharmaceutical ingredient a) does not comprise idelalisib.

Caffeine is a compound of the following formula (1)

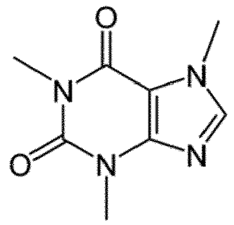

(1)

and has the IUPAC name 1,3,7-trimethyl-1H-purine-2,6(3H,7H)-dione 3,7-dihydro-1,3,7-trimethyl-1H-purine-2,6-dione. Caffeine is reported to exist in two polymorphic forms, a metastable form (mod. I or α-form) and the enatiotropically related stable form (mod. II or β-form).

Indomethacin is a non-steroidal anti-inflammatory drug (NSAID) commonly used as a prescription medication to reduce fever, pain, stiffness and swelling. It works by inhibiting the production of prostaglandins, molecules known to cause these symptoms. It is a compound of the following formula (2)

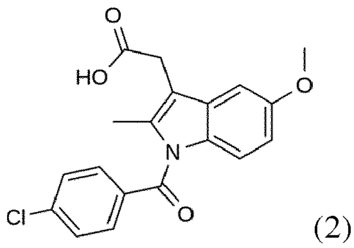

(2)

and has the IUPAC name 2-{1-[(4-chlorophenyl)carbonyl]-5-methoxy-2-methyl-1H-indol-3-yl}acetic acid. Indomethacin exists in three polymorphs, named α, γ and δ, as well as in an amorphous form. At room temperature, by pressurization from atmospheric pressure, γ-indomethacin undergoes a collapse transformation into a high-pressure crystalline form, induced by large rearrangement in the hydrogen-bonded network associated with molecular conformational changes. Upon further compression a solid-state amorphization is observed via the breakdown of hydrogen bonds.

Sulfabenzamide is an antibacterial/antimicrobial with the following formula (3)

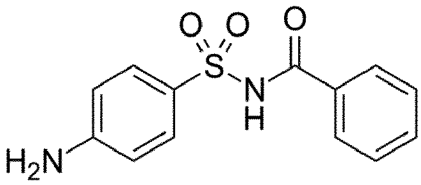

(3)

and has the IUPAC name 4-amino-N-benzoyl-benzenesulfonamide. Sulfabenzamide exists in a stable Form A, having a melting point of 181°C, and an unstable Form B, having a melting point of 171°C, wherein upon compression Form B transforms into Form A.

Paroxetine is an antidepressant drug of the SSRI type. Paroxetine is used to treat major depression, obsessive-compulsive disorder, panic disorder, social anxiety, posttraumatic stress disorder, generalized anxiety disorder and vasomotor symptoms (e.g. hot flashes and night sweats) associated with menopause in adult outpatients. It is a compound of the following formula (4)

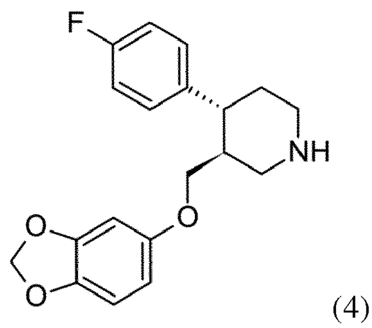

(4)

and has the IUPAC name (3S,4R)-3-[(2H-1,3-benzodioxol-5-yloxy)methyl]-4-(4-fluorophenyl)piperidine. Paroxetine hydrochloride exists in two polymorphic forms, a Form I and a Form II, wherein Form II transforms into Form I upon compression.

Theophylline, also known as 1,3-dimethylxanthine, is a methylxanthine drug used in therapy for respiratory diseases such as chronic obstructive pulmonary disease (COPD) and asthma under a variety of brand names. As a member of the xanthine family it bears structural and pharmacological similarity to caffeine. Theophylline is a compound of the following formula (5)

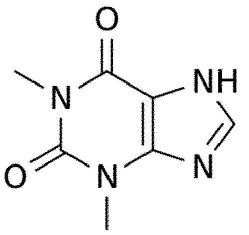

(5)

and has the IUPAC name 1,3-dimethyl-7H-purine-2,6-dione. Theophylline is known to exist as a crystalline monohydrate and four anhydrous polymorphs (Form I, Form II, Form III and Form IV).

Amoxicillin is an antibiotic useful for the treatment of a number of bacterial infections, including acute otitis media, streptococcal pharyngitis, pneumonia, skin infections, urinary tract infections, Salmonella infections, Lyme disease and chlamydia infections. It is a moderate-spectrum, bacteriolytic, β-lactam antibiotic in the aminopenicillin family used to treat susceptible Gram-positive and Gram-negative bacteria. It is usually the drug of choice within the class because it is better-absorbed, following oral administration, than other β-lactam antibiotics. Amoxicillin is susceptible to degradation by β-lactamase-producing bacteria, which are resistant to a narrow spectrum of β-lactam antibiotics such as penicillin. For this reason, it is often combined with clavulanic acid, a β-lactamase inhibitor. This increases effectiveness by reducing its susceptibility to β-lactamase resistance. Amoxicillin is a compound of the following formula (6)

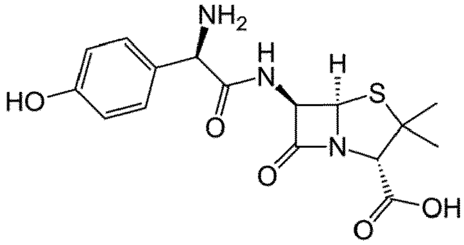

(6)

and has the IUPAC name (2S,5R,6R)-6-{[(2R)-2-amino-2-(4-hydroxyphenyl)-acetyl]amino}-3,3-dimethyl-7-oxo-4-thia-1-azabicyclo[3.2.0]heptane-24-carboxylic acid.

Cephalexin is an antibiotic useful for the treatment of a number of bacterial infections including: middle ear infections, strep throat, bone and joint infections, pneumonia, skin infections and urinary tract infections. It is active against Gram-positive bacteria and some Gram-negative bacteria. It is ineffective against methicillin-resistant Staphylococcus aureus. It is in the class of first generation cephalosporins and has similar activity to other agents within this group including the intravenous agent cefazolin. Cephalexin is a compound of the following formula (7)

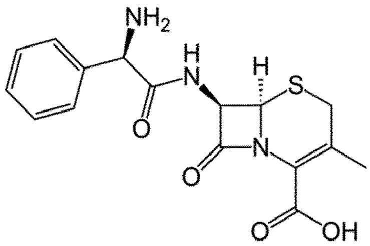
(7)

and has the IUPAC name (7R)-3-methyl-7- (α-D-phenylglycylamino)-3-cephem-4-carboxylic acid monohydrate. Cephalexin is known to exist in an amorphous form and as crystalline monohydrate.

Cefixime is an antibiotic useful for the treatment of a number of bacterial infections including otitis caused by *Haemophilus influenzae, Moraxella catarrhalis* and *Streptococcus pyogenes,* Sinusitis, tonsillitis, pharyngitis caused by *Streptococcus pyogenes,* bronchitis, pneumonia caused by *Streptococcus pneumoniae* and *Haemophilus influenzae.* It is a third generation cephalosporin. Cefixime is a compound of the following formula (8)

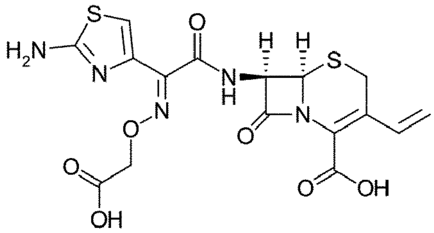
(8)

and has the IUPAC name (6R,7R)-7-{[2-(2-amino-1,3-thiazol-4-yl)-2-(carboxymethoxyimino)acetyl]amino}-3-ethenyl-8-oxo-5-thia-1-azabicyclo[4.2.0]oct-2-ene-2-carboxylic acid. Cefixime is known to exist in an amorphous form and as crystalline trihydrate.

Piroxicam is a non-steroidal anti-inflammatory drug (NSAID) of the oxicam class used to relieve the symptoms of painful inflammatory conditions like arthritis, rheumatoid and osteoarthritis, primary dysmenorrhoea, postoperative pain. It also acts as an analgesic, especially where there is an inflammatory component. Piroxicam belongs to the biopharmaceutical classification system class II drugs of low aqueous solubility and high permeability. It is a compound of the following formula (9)

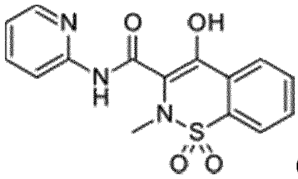
(9)

and has the IUPAC name 4-hydroxy-2-methyl-N-(2-pyridinyl)-2H-1,2-benzothiazine-3-carboxamide 1,1-dioxide. Crystalline piroxicam is polymorphic and exists in various crystalline forms designated as Form I, Form II, Form III, and monohydrate form. Form I is also named β, cubic form, and Form A; meanwhile Form II is termed α, α1, α2, needle form, and Form B. Form III is thermally unstable and can be converted to Form II and Form I, respectively. Additionally, the monohydrate form can dehydrate to Form I. Piroxicam Form II can transform to Form I by pressure (Ghan G. A., Lalla J. K., Effect of compressional forces on piroxicam polymorphs. J. Pharm. Pharmacol. 1992;44:678-681).

Acetaminophen (also known as paracetamol) is a widely used over-the-counter analgesic (pain reliever) and antipyretic (fever reducer). Though acetaminophen is used to treat inflammatory pain, it is not generally classified as an NSAID because it exhibits only weak anti-inflammatory activity. Acetaminophen is a compound of the following formula (10)

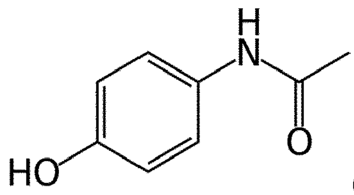

(10)

and has the IUPAC name N-(4-hydroxyphenyl)ethanamide. Acetaminophen can be amorphous or crystalline and has three polymorphs: stable Form I (monoclinic), metastable Form II (orthorhombic) and unstable form III. Below -120°C, the Form II polymorph becomes more stable than Form I. Form II tends to transform to Form I under pressure.

Chlorpropamide is a drug in the sulfonylurea class used to treat type 2 diabetes mellitus. It is a long-acting first generation sulfonylurea. Chlorpropamide is a compound of the general formula (11)

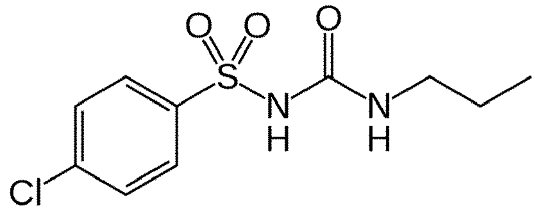

(11)

and has the IUPAC name 4-chloro-N-(propylcarbamoyl)benzenesulfonamide. Chlorpropamide is known to exhibit at least six polymorphic phases. These forms are characterized not only by variations in their molecular packing but also in their molecular conformation. According to Burger's nomenclature, the polymorphs are labeled I, II, III, IV, and V, with Form III being the commercial one. At this point, it is worthwhile to cite that Forms I, II, and III are usually referred to as C, B, and A, respectively, due to the wide diffusion of the nomenclature defined by Simmons et al. (D. L. Simmons, R. J. Ranz, N. D. Gyanchandani, Can. J. Pharm. Sci. 1973, 8, 125). On tabletting, Form A has been reported to transform partially into another polymorph, Form C.

Idelalisib is a drug suitable for the treatment of chronic lymphocytic leukaemia. It is in Phase III clinical trials testing drug combinations with rituximab and/or bendamustine. The substance acts as a phosphoinositide 3-kinase inhibitor; more specifically, it blocks P110δ, the delta isoform of the enzyme phosphoinositide 3-kinase. Idelalisib is a compound of the general formula (12)

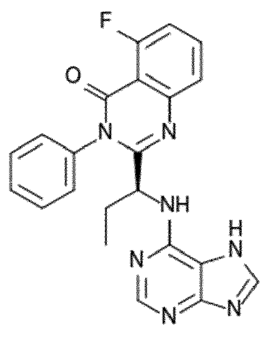

(12)

and has the IUPAC name 5-fluoro-3-phenyl-2-[(1S)-1-(7H-purin-6-ylamino)propyl]-4(3H)-quinazolinone. Idelalisib is known to exist in at least seven polymorphic forms. In one embodiment, idelalisib is present in the dosage form of the present invention as polymorphic Form I which exhibits an XRPD pattern (measured as described above) substantially as shown in FIG. 1A of WO 2013/134288. Preferably, Form I of idelalisib has an X-ray powder diffraction pattern that includes characteristic peaks at about 17.7° 2θ and about 24.9° 2θ. In some embodiments, the X-ray powder diffraction pattern further includes any one or more characteristic peaks at about 14.3° 2θ, about 17.2° 2θ, about 20.9° 2θ and about 23.9° 2θ. In some embodiments, the polymorphic Form I has a melting temperature of about 254°C to about 256°C. In one variation, polymorphic Form I has an X-ray powder diffraction pattern that includes any one or more characteristic peaks at about 14.3° 2θ, about 17.2° 2θ, about 17.7° 2θ, about 20.9° 2θ, about 23.9° 2θ and about 24.9° 2θ; and a melting temperature of about 254 °C to about 256°C (cf. [0009] of WO 2013/134288). Polymorph Form I of

idelalisib is described in and can be obtained according to WO 2013/134288.

Polymorphic Form I of idelalisib converts under pressure conditions which are typical for tablet preparation at least partially to a polymorphic Form II which exhibits an XRPD pattern (measured as described above) substantially as shown in FIG. 2A of WO 2013/134288.

Preferably, Form II has an X-ray powder diffraction pattern that includes a characteristic peak at about 18.6° 2θ. In some embodiments, the X-ray powder diffraction pattern further includes characteristic peaks at about 24.3° 2θ and about 14.0° 2θ (cf. [0028] of WO 2013/134288).

The pressure sensitive solid form of an active pharmaceutical ingredient can preferably be present in amounts of 5 up to 75 wt.%, preferably of 20 to 74 wt.%, in particular of 40 to 70 wt.% based on the total weight of the dosage form. The pressure sensitive solid form of an active pharmaceutical ingredient can preferably be present in the dosage form in amounts of 1 to 800 mg, such as 1, 2, 3, 4, 5, 6, 7, 8, 9, 10, 50, 100, 200, 300, 400, 500, 600, 700, 750 mg, preferably of 10 to 500, such as 10, 15, 20, 25, 30, 35, 40, 45, 50, 60, 70, 80, 90, 100, 150, 200, 250, 300, 350, 400, 450 mg, in particular of 100 to 300 mg, such as 110, 120, 130, 140, 150, 160, 170, 180, 190, 200, 210, 220, 230, 240, 250, 260, 270, 280, 290, 300 mg, based on the amount of the active pharmaceutical ingredient in form of the free base/acid. If the active ingredient is present in the form of a salt, the amount of the corresponding acid/base has to be added accordingly. The active pharmaceutical ingredient comprised in the dosage form of the present invention can preferably have an average particle size (D50) of 0.05 to 250 μm, more preferably 0.1 to 50 μm, even more preferably 1 to 45 μm, especially 2 to 20 μm. The average particle size (D50), which is also denoted as the D50 value of the integral volume distribution, is defined in the context of this invention as the particle diameter at which 50 per cent by volume of the particles have a smaller diameter than the diameter which corresponds to the D50 value. Likewise, 50 per cent by volume of the particles have a larger diameter than the D50 value.

The active pharmaceutical ingredient comprised in the dosage form of the present invention can preferably have a particle size (D10) of 0.02 to 80 μm, more preferably 0.3 to 20 μm, even more preferably 0.4 to 15 μm, especially preferably 0.5 to 5 μm. The particle size (D10), which is also denoted as the D10 value of the integral volume distribution, is defined in the context of this invention as the particle diameter at which 10 per cent by volume of the particles have a smaller diameter than the diameter which corresponds to the D10 value. Likewise, 90 per cent by volume of the particles have a larger diameter than the D10 value.

The active pharmaceutical ingredient comprised in the dosage form of the present invention can preferably have an average particle size (D90) of 0.2 to 600 μm, more preferably 0.5 to 150 μm, even more preferably 3 to 120 μm, especially 7 to 55 μm. The particle size (D90), which is also denoted as the D90 value of the integral volume distribution, is defined analogously to D10 and D50.

The particle size distribution (by volume) may be measured using a laser diffraction sizing instrument, for example the Sympatec Helos device (available from Sympactec GmbH, Germany) using the Cuvette dispersion device.

In order to carry out the measurement, a stock dispersion may be prepared by mixing the active pharmaceutical ingredient with a dispersing aid (e.g. paraffine oil) until a uniform paste is formed. The paste may then be diluted and mixed to a final volume of 3 to 6 ml using white spirit. The optical concentration of the final solution should remain below 5%. The percent values are calculated from the mean cumulative volume size curve by the software of the Sympatec instrument. D90 means 90% of the particle size population is below the specified value in volume. D50 and D10 are defined accordingly.

The dosage form of the present invention further comprises a first disintegrant b) having elastic properties.

Disintegrants are compounds which can enhance the ability of a dosage form to break into smaller fragments when in contact with a liquid, preferably water. A disintegrant having elastic properties is understood to be a disintegrant which deforms mainly in an elastic manner when a force is applied. Elastic deformation is a reversible deformation. Once the force is no longer applied, the object returns to its original shape. Without being bound to theory, it is suggested that due to its elastic properties, the disintegrant can absorb the compression energy during the compression to obtain the compressed dosage form of the present invention and prevents the pressure sensitive solid form of the active pharmaceutical ingredient from at least partially converting in another form.

Preferably, the first disintegrant b) forms a two- or three-dimensional network or matrix surrounding particles of the pressure sensitive solid form of the active pharmaceutical ingredient. Upon compression, the network absorbs the compression energy, thus protecting the pressure sensitive solid form of the active pharmaceutical ingredient.

In one embodiment of the invention, the first disintegrant b) has a Brittle Fracture Index (BFI) of less than 0.15, preferably 0.05 to 0.15, more preferably 0.09 to 0.13.

The Brittle Fracture Index (BFI) is a measure of brittleness of a material and the ability of a compact to relieve stress by elastic-plastic deformation. The Brittle Fracture Index is determined by comparing the tensile strength of a compact, sT, to that of a compact with a small hole (stress concentrator) in it, $sT_0$, using the tensile test as described below. A hole in the centre of the compact generally weakens a tablet. If a material is very brittle, theoretical considerations show that the tensile strength of a tablet with a hole in it will be about 1/3 that of a "solid" tablet. The Brittle Fracture Index is defined according to Calculation Formula (1):

$$BFI = [(sT / sT_0) - 1] \cdot 0.5 \qquad \text{(Calculation Formula (1))}$$

For non-brittle materials, stress can be relieved by elastic-plastic deformation and the tensile strength of the two compacts will be more similar. The BFI may approach zero for very non-brittle materials and approach 1 for very brittle materials.

The tensile strength, sT, of square compacts is determined by measuring the force necessary to cause tensile failure under traverse compression (E. Hiestand, C. Peot, Tensile strength of compressed powders and an example of incompatibility as endpoint on shear yield locus. J Pharm Sci 1974; 63: 605-612). The transverse compression of square compacts between platens narrower than the compact produces tensile stresses at the centre of the compact and provides a highly reproducible method of evaluating the tensile strength of the compacts of powdered materials.

For measuring the tensile strength and, consequently, deriving the BFI values therefrom, a simple hydraulic jack press is fitted with platens 0.4 times the width of the square tablet. Pads from ink blotters are used on the platens and the tablet is centred on edge between the platens. The jack is equipped with a strain gauge transducer to indicate the magnitude of the force of fracture. The hydraulic fluid is supplied from the compression of a smaller cylinder that is operated by a motorized screw jack. The constant rate of compression yields an exponential load application with a time constant of 15 s. The apparatus is shown schematically in FIG. 1. Tablets are made for the BFI test with a hole in their centre by using a special punch that has a spring-loaded retractable (into the punch) pin. The square compacts are 38.1 mm on a side and when a hole is present it has a diameter of 1.1 mm. Production of tablets and measuring BFI is performed under standard environmental air conditions which is room temperature (25°C) and relative humidity of 50%.

Preferably, the first disintegrant b) having elastic properties is selected from a cross-linked cellulose derivative, such as croscarmellose sodium, or non cross-linked cellulose derivative, such as hydroxypropyl cellulose, hydroxypropyl methylcellulose and methylcellulose. Preferably, the first disintegrant b) having elastic properties is a cross-linked cellulose derivative.

More preferably, the first disintegrant b) is croscarmellose sodium (e.g. Ac-Di-Sol®, Primellose®). Preferably, croscarmellose sodium complies with the specification of Ph. Eur. 7.0 (monograph 0985). Croscarmellose sodium is a cross-linked polymer of carboxymethyl cellulose sodium. Crosslinking makes it an insoluble, hydrophilic, highly absorptive material, resulting in excellent swelling properties and its unique fibrous nature gives excellent water wicking capabilities. Water wicking (capillary action) is the ability to draw water into the tablet matrix. Exposure to water can cause ingredients to swell and exert pressure against the surrounding tablet or capsule ingredients, causing existing bonds between particles to break.

Preferably, the sodium croscarmellose has a degree of substitution in the range of 0.3 to 1.0, more preferably in the range of 0.5 to 0.9, most preferably 0.6 to 0.85. A substitution degree of 1.0 means an average of 10 carboxymethyl groups per 10 anhydroglucose units. The degree of substitution can be determined according to Ph. Eur. 7.0 (monograph 0985). Croscarmellose having various degrees of substitution are commercially available.

The first disintegrant b) can preferably be present in amounts of 1 up to 25 wt.%, preferably of 1.5 to 20 wt.%, in particular of 5 to 15 wt.% based on the total weight of the dosage form.

In one embodiment, the first disintegrant b) has a median particle size, as measured by laser light diffraction, in the range of 20 to 150 μm, preferably 30 to 100 μm, more preferably 40 to 70 μm.

In one embodiment, the weight ratio of the pressure sensitive solid form of an active pharmaceutical ingredient a) to the first disintegrant having elastic properties is from 10:1 to 1:7, such as 10:1, 9:1, 8:1, 7:1, 6:1, 5:1, 4:1, 3:1, 8:3, 5:2, 2:1, 3:2, 4:3, 1:1, 3:4, 2:3, 1:2, 2:5, 8:3, 1:3, 3:10, 1:4, 3:14, 1:5 or 1:6. More preferably the weight ratio of the pressure sensitive solid form of an active pharmaceutical ingredient a) to the first disintegrant having elastic properties is from 3:1 to 1:6, such as 8:3, 5:2, 2:1, 3:2, 4:3, 1:1, 3:4, 2:3, 1:2, 2:5, 8:3, 1:3, 3:10, 1:4, 3:14 or 1:5. Most preferably the weight ratio of the pressure sensitive solid form of an active pharmaceutical ingredient a) to the first disintegrant having elastic properties is from 5:1 to 1:5, such as 1:1, 4:3, 3:4, 3:2, 2:3, 2:1, 1:2, 5:2, 2:5, 3:8, 8:3, 3:1, 1:3, 10:3, 3:10, 4:1 or 1:4.

The pressure sensitive solid form of an active pharmaceutical ingredient a) and the first disintegrant having elastic properties b) are preferably associated with one another, e.g. prior to compression, in order for the first disintegrant b) to effectively absorb energy during the compression step of the production process of a compressed solid dosage form of the present invention,

The association may be carried out in different forms. In one embodiment, the pressure sensitive solid form of an active pharmaceutical ingredient a) and the first disintegrant having elastic properties b) may be associated with one another in a form as obtained by direct compression. Preferably, the pressure sensitive solid form of an active pharmaceutical ingredient a), the first disintegrant having elastic properties b), the second disintegrant c) and optionally the further pharmaceutical excipient d) are mixed together, e.g. in a free-fall mixer, and directly compressed.

In a preferred embodiment, the pressure sensitive solid form of an active pharmaceutical ingredient a) and the

first disintegrant having elastic properties b) may be associated with one another in form of granules. Preferably, the granules are formed by dry or wet granulation, more preferably the granules are formed by wet granulation, e.g. prior to compression.

In one preferred embodiment, the granules comprise the pressure sensitive solid form of an active pharmaceutical ingredient a) and the first disintegrant having elastic properties b), while the second disintegrant c) and the further pharmaceutical excipient d) are not comprised in the granules, i.e. they are in the phase between the granules (or external phase).

In a further preferred embodiment, the granules comprise the pressure sensitive solid form of an active pharmaceutical ingredient a), the first disintegrant having elastic properties b) and at least a first part of the further pharmaceutical excipient d), while the second part of the further pharmaceutical excipient d) and the second disintegrant c) are not comprised in the granules, i.e. they are in the phase between the granules. For example, the granules can comprise one or more components d1) to d6) (see below), e.g. a filler d1) and binder d3), while one or more of the components d2) and d4) to d6) are not comprised in the granules, i.e. they are either not present in the dosage form or they are in the external phase. Alternatively, a first part of one or more components d1) to d6), for example a first part of filler d1), such as a first half of the total amount of filler d1) present in the dosage form, is comprised in the granules, while a second part of the one or more components d1) to d6), for example a second part of filler d1), such as the second half of the total amount of filler d1) present in the dosage form, is outside the granules, that is in the external phase.

Preferably, the granules are obtainable by wet granulating the pressure sensitive solid form of an active pharmaceutical ingredient a), the first disintegrant having elastic properties b) and optionally the further pharmaceutical excipient d). As described above, when the further pharmaceutical excipient d) is wet granulated with the pressure sensitive solid form of an active pharmaceutical ingredient a) and the first disintegrant b), it may comprise one or more of components d1) to d6) or a first part of one or more components d1 to d6).

"Granulating" is generally understood to mean the formation of granular aggregate material as a powder by assembling and/or aggregating finer powder particles and/or the formation of finer granules by breaking up coarser aggregates.

"Wet granulation" means a granulation with a granulation liquid. A mixture comprising a pressure sensitive solid form of an active pharmaceutical ingredient a), a first disintegrant having elastic properties b) and optionally a further pharmaceutical excipient d) is preferably wetted with the granulation liquid. Preferably, the wet granulation is carried out in the presence of a binder d3), in particular hydroxypropyl cellulose, hydroxypropyl methylcellulose or polyvinylpyrrolidone. Suitable liquids for preparing the granulation liquid are, for example, water, alcohols, such as isopropanol and 1-propanol, and mixtures thereof. A preferred granulation liquid is water.

According to an embodiment of the present invention, the compressed solid dosage form further comprises a second disintegrant c). Preferably the first disintegrant b) and the second disintegrant c) are different compounds.

Preferably, the second disintegrant c) is selected from starch, starch derivatives, cellulose, cellulose derivatives, polyvinylpyrrolidone, respectively either cross-linked or not cross-linked, and mixtures thereof.

Preferably, the second disintegrant is selected from partially pregelatinized (maize) starch, sodium starch glycolate, cross-linked polyvinylpyrrolidone (crospovidone), carboxymethyl cellulose, hydroxypropyl cellulose (HPC), hydroxypropyl methylcellulose, methylcellulose and mixtures thereof. More preferably, the second disintegrant is selected from sodium starch glycolate, cross-linked polyvinylpyrrolidone (crospovidone) and mixtures thereof.

The second disintegrant c) can preferably be present in amounts of 1 up to 15 wt.%, preferably of 1.5 to 8 wt.%, in particular of 2 to 5 wt.% based on the total weight of the dosage form.

The dosage form according to the present invention optionally contains a further pharmaceutical excipient d). As defined herein, the term "pharmaceutical excipient" comprises the single as well as the plural form, i.e. the dosage form of the present invention may comprise one or more further pharmaceutical excipients. These are pharmaceutical excipients with which the person skilled in the art is familiar, such as those which are described in the European Pharmacopoeia and/or in the US Pharmacopoeia.

In a preferred embodiment, the pharmaceutical excipient d) can preferably be selected from fillers d1), glidants d2), binders d3), lubricants d4), sweeteners d5), flavours d6) and mixtures thereof.

The pharmaceutical excipient d) can preferably be present in amounts of 0 up to 50 wt.%, preferably of 0.5 to 40 wt.%, in particular of 1 to 25 wt.% based on the total weight of the dosage form.

Fillers d1) add volume and/or mass to a drug substance, thereby facilitating precise metering and handling thereof in the preparation of dosage forms. Fillers typically also fill out the size of a tablet or capsule, making it practical to produce and convenient for the consumer to use. A filler is generally inert, compatible with the other components of the formulation, non-hygroscopic, relatively cheap, compactible, and preferably tasteless or pleasant tasting.

In a preferred embodiment the filler is referred to as a single filler or to a mixture of several fillers.

It is preferred that the filler is provided in an amount of 0 to 40 wt.%, preferably 0.2 to 30 wt.%, more preferably 0.5 to 15 wt.%, based on the total weight of the dosage form.

In an embodiment of the invention, the filler can be selected from magnesium carbonate, magnesium aluminosilicates, calcium carbonate, calcium phosphate, calcium hydrogen phosphate, calcium dihydrogen phosphate, a sugar alcohol, such as erythritol, isomaltose, mannitol, maltitol, sorbitol, threitol and xylitol, a monosaccharide, such as arabinose, galactose, glucose, mannose and xylose, a disaccharide, such as isomaltose, lactose, maltose, sucrose, an oligosaccharide, such as cyclodextrin, maltodextrin and raffinose, a polysaccharide, such as cellulose (e.g. microcrystalline cellulose), glycogen and starch.

In a further preferred embodiment, the filler can preferably be selected from microcrystalline cellulose, lactose, mannitol and starch, more preferably from lactose and microcrystalline cellulose.

Glidants d2) can be used to improve the flowability. Preferably, the glidant is talc or colloidal silica (e.g. Aerosil®), more preferably colloidal silica. Preferably, the glidant can be present in an amount of 0 to up to 3 wt.%, more preferably of 0.1 to 2.8 wt.%, in particular of 0.2 to 2.5 wt.% based on the total weight of the dosage form.

Binders d3) should be capable of ensuring that granules or tablets can be formed with the required mechanical strength. Binders can be, for example, gelatine, polyvinylpyrrolidone, cellulose derivatives, such as hydroxypropyl methylcellulose and hydroxypropyl cellulose. Binders can preferably be present in amounts of 0 up to 10 wt.%, preferably of 0.2 to 8 wt.%, in particular of 0.3 to 6.0 wt.%, based on the total weight of the dosage form.

Lubricants d4) are generally used in order to reduce sliding friction. Suitable lubricants are, for example, stearic acid, adipic acid, sodium stearyl fumarate and/or magnesium stearate, preferably magnesium stearate. Preferably, lubricants can be present in an amount of 0 to up to 5 wt.%, more preferably of 0.1 to 4 wt.%, in particular of 0.2 to 2.5 wt.% based on the total weight of the dosage form.

Sweeteners d5) are added to make the ingredients more palatable. Suitable sweeteners are, for example, sorbitol, dextrose, sucrose, saccharin, aspartame, acesulfame-K and sucralose. Preferably, sweeteners can be present in an amount of 0 to up to 10 wt.%, more preferably of 0.2 to 8 wt.%, in particular of 0.5 to 6 wt.% based on the total weight of the dosage form.

Flavours d6) can be used to mask unpleasant taste of the active ingredients and increase the probability that the patient will complete a course of medication. The U.S. Code of Federal Regulations describes a natural flavorant as: an essential oil, oleoresin, essence or extractive, protein hydrolysate, distillate or any product of roasting, heating or enzymolysis which contains the flavoring constituents derived from a spice, fruit or fruit juice, vegetable or vegetable juice, edible yeast, herb, bark, bud, root, leaf or similar plant material, dairy products or fermentation products thereof whose significant function in food is flavoring rather than nutritional.

Artificial flavors include any substance the function of which is to impart flavor which is not derived from a spice, fruit or fruit juice, vegetable or vegetable juice, edible yeast, herb, bark, bud, root, leaf or similar plant material, meat, fish, poultry, eggs, dairy products or fermentation products thereof.

Preferably, flavours can be present in an amount of 0 to up to 5 wt.%, more preferably of 0.2 to 4 wt.%, in particular of 0.3 to 3 wt.% based on the total weight of the dosage form.

As it cannot be excluded that a certain compound meets the requirements of more than one of the excipients described above, in the context of the present invention a compound which is used as a first excipient is not simultaneously also used as a second excipient.

In a preferred embodiment, the compressed solid dosage form according to the invention provides an immediate release of the active pharmaceutical ingredient a). This means that the release profile of the dosage forms of the invention according to USP app. II (paddle, 900 ml, 0.1 M HCl, 50 rpm, 37°C) after 15 minutes preferably indicates a content release of the active pharmaceutical ingredient of 70% to 100%, preferably more than 75%, especially more than 80%. For this embodiment, the active pharmaceutical ingredient is preferably selected from BCS class II and IV.

In a preferred embodiment, the dosage form of the present invention can preferably comprise the following amounts of components:

a) 5 to 75 wt.-% pressure sensitive solid form of an active pharmaceutical ingredient,
b) 1 to 25 wt.-% first disintegrant,
c) 1 to 15 wt.-% second disintegrant,
d) 0 to 55 wt.% pharmaceutical excipient,

based on the total weight of the dosage form. For this embodiment, the active pharmaceutical ingredient is preferably selected from BCS class II and IV.

In a preferred embodiment, the dosage form of the present invention can preferably comprise the following amounts of components:

a) 5 to 75 wt.-%, preferably 20 to 74 wt.%, in particular 40 to 70 wt.% pressure sensitive solid form of an active pharmaceutical ingredient,
b) 1 to 25 wt.-%, preferably 1.5 to 20 wt.%, in particular 5 to 15 wt.% first disintegrant,
c) 1 to 15 wt.-%, preferably 1.5 to 8 wt.%, in particular 2 to 5 wt.% second disintegrant,

d1) 0 to 40 wt.-%, preferably 0.2 to 30 wt.-%, in particular 0.5 to 30 wt.-% filler,
d2) 0 to 3 wt.-%, preferably 0.1 to 2.8 wt.-%, in particular 0.2 to 2.5 wt.-% glidant,
d3) 0 to 10 wt.-%, preferably 0.2 to 8 wt.-%, in particular 0.3 to 6 wt.-% binder,
d4) 0 to 5 wt.-%, preferably 0.1 to 4 wt.-%, in particular 0.2 to 2.5 wt.-% lubricant,
d5) 0 to 10 wt.-%, preferably 0.2 to 8 wt.%, in particular 0.5 to 6 wt.% sweetener,
d6) 0 to 5 wt.-%, preferably 0.2 to 4 wt.%, in particular 0.3 to 3 wt.% flavour, based on the total weight of the dosage form. For this embodiment, the active pharmaceutical ingredient is preferably selected from BCS class II and IV.

In a preferred embodiment, the dosage form of the present invention can preferably comprise the following amounts of components:

a) 5 to 75 wt.-% pressure sensitive solid form of an active pharmaceutical ingredient,
b) 1 to 25 wt.-% croscarmellose sodium,
c) 1 to 15 wt.-% partially pregelatinized (maize) starch, sodium starch glycolate, cross-linked polyvinylpyrrolidone (crospovidone), carboxymethyl cellulose, hydroxypropyl cellulose (HPC), hydroxypropyl methylcellulose, methylcellulose or mixtures thereof,
d1) 0 to 40 wt.-% microcrystalline cellulose, glucose, lactose, mannitol, starch or mixtures thereof
d2) 0 to 3 wt.-% colloidal silica,
d3) 0 to 10 wt.-% gelatine, polyvinylpyrrolidone, hydroxypropyl methylcellulose, hydroxypropyl cellulose or mixtures thereof
d4) 0 to 5 wt.-% stearic acid, adipic acid, sodium stearyl fumarate, magnesium stearate, or mixtures thereof,

based on the total weight of the dosage form. For this embodiment, the active pharmaceutical ingredient is preferably selected from BCS class II and IV.

In a preferred embodiment, the dosage from of the present invention can preferably comprise the following amounts of components:

a) 1 to 800 mg pressure sensitive solid form of an active pharmaceutical ingredient,
b) 0.5 to 500 mg first disintegrant,
c) 0.5 to 350 mg second disintegrant,
d1) 0 to 600 mg filler,
d2) 0 to 100 mg glidant,
d3) 0 to 100 mg binder,
d4) 0 to 80 mg lubricant.

In a preferred embodiment, the dosage from of the present invention can preferably comprise the following amounts of components:

a) 50 to 350 mg Form I of idelalisib,
b) 10 to 60 mg first disintegrant,
c) 1 to 15 mg second disintegrant,
d1) 5 to 40 mg filler,
d2) 1 to 12 mg glidant,
d3) 0 to 20 mg binder,
d4) 1 to 10 mg lubricant.

In a preferred embodiment, the dosage from of the present invention can preferably comprise the following amounts of components:

a) 50 to 350 mg Form I of idelalisib,
b) 10 to 60 mg croscarmellose sodium,
c) 1 to 15 mg sodium starch glycolate,
d1) 5 to 40 mg microcrystalline cellulose or lactose,
d2) 1 to 12 mg colloid silica,
d3) 0 to 20 mg polyvinylpyrrolidone or hydroxypropyl cellulose,
d4) 1 to 10 mg magnesium stearate.

In a further embodiment of the compressed solid dosage form of the present invention, the dosage form can

optionally comprise a coating e). For this purpose, the methods of film-coating dosage forms such as tablets as standard in the state of the art may be employed. The above-mentioned amounts of active pharmaceutical ingredient, disintegrants and excipient, however, relate to the uncoated dosage form.

Typical film-coatings are preferably formed using polymers such as modified celluloses, polyvinyl acetate phthalate, polyvinylpyrrolidone, polymethacrylates and/or shellack.

In another preferred embodiment, the dosage form is a tablet.

The tablet may be a tablet which can be swallowed unchewed. It may likewise be a chewable tablet or a dispersible tablet. In the case of tablets which are swallowed unchewed, it is preferable that they be coated with a film layer.

Further, the present invention relates to a process for the production of a compressed solid dosage form comprising a pressure sensitive solid form of an active pharmaceutical ingredient comprising the steps of

I) providing a pressure sensitive solid form of an active pharmaceutical ingredient a), a first disintegrant having elastic properties b) and optionally a further pharmaceutical excipient d),
II) mixing, and preferably granulating, the mixture of step I), preferably with a granulation liquid, and
III) optionally drying the granules from step II),

wherein a pressure sensitive solid form is defined as a form which modifies its degree of structural order under a pressure of 30 MPa.

In step I) according to the present invention, a pressure sensitive solid form of an active pharmaceutical ingredient a), a first disintegrant having elastic properties b) and optionally a further pharmaceutical excipient d) are provided.

The pressure sensitive solid form of an active pharmaceutical ingredient a), the first disintegrant having elastic properties b) and optionally a further pharmaceutical excipient d) are defined as above. Hence, the pharmaceutical excipient d) comprises fillers d1), glidants d2), binders d3), lubricants d4), sweeteners d5) and flavours d6).

In step II), the mixture of step I) is mixed and preferably granulated, preferably with a granulation liquid.

In one embodiment, the pressure sensitive solid form of an active pharmaceutical ingredient a), the first disintegrant having elastic properties b) and, optionally, a further pharmaceutical excipient d) can be mixed or blended in order to provide a composition with a homogenous distribution of the active pharmaceutical ingredient a) within the resulting blend, comprising active pharmaceutical ingredient a), first disintegrant b) and, optionally, a further pharmaceutical excipient d). Mixing can be carried out with conventional mixing devices such as a free-fall mixer.

In an alternative embodiment, the mixing can be conducted such that the active pharmaceutical ingredient a) is mixed with a first part of the first disintegrant b) and optionally a further pharmaceutical excipient d) in a mixing device, for example in a high shear or tumbler mixer. After this first mixing step, a second part of the first disintegrant b) and optionally the further pharmaceutical excipient d) can be added, which step is followed by a second mixing step. This procedure can be repeated until the last part of the first disintegrant b) and optionally the further pharmaceutical excipient d) is added, preferably one to five times.

Further in step II), the blended mixture is preferably granulated, preferably with a granulation liquid. Therefore, in step II) the mixture of step I) is blended and optionally the resulting blend is granulated.

The blended mixture from step I) is preferably wetted with a granulation liquid. Preferably, the granulation liquid comprises at least one further pharmaceutical excipient d), preferably a binder d3), in particular hydroxypropyl methylcellulose, hydroxypropyl cellulose or polyvinylpyrrolidone. Suitable liquids for preparing the granulation liquid are, for example, water, alcohols, such as 1-propanol and isopropanol, and mixtures thereof. A preferred granulation liquid is water. In step III), the granules from step II) are optionally dried. Preferably, step III) is carried out for granules formed by wet granulation in step II).

In an embodiment, the granulation is carried out for 1 to 60 minutes, preferably 2 to 30 minutes.

The wet granulation process can for example be carried out in high-speed mixers/granulators, fluidized-bed granulators, a spray drier or according to the pellet-layering method.

In a further embodiment, in step II) the blended mixture is granulated without a granulation liquid. This process is called dry granulation.

In dry granulation, the blended mixture of step I) is compacted by applying a force onto the powder.

Dry granulation can be for example carried out by slugging or roller compaction.

The term slugging is generally used when a tablet press is used for the compaction process.

Preferably, dry granulation is carried out by roller compaction. A roller compactor generally consist of three major units: A feeding system, which conveys the powder to the compaction area between the rolls; a compaction unit, where powder is compacted between two counter rotating rolls to a ribbon by applying a force; a size reduction unit, for milling the ribbons to the desired particle size. For dry granulated granules a drying step III) is not necessary.

Preferably, the blended mixture of step I) is granulated in step II) by wet granulation.

In a further embodiment, after mixing the mixture of step I), step IV) is carried out without granulating and drying the blended mixture from step II). In a preferred embodiment, the blended mixture from step II) can be sieved before

being blended in step IV).

If wet granulation is performed, a drying step III) can preferably be employed. "Drying" means the separation of liquids adhering to solids. Drying generally takes place in conventional drying equipment, such as fluidised-bed dryers, freeze dryers, tray dryers, spray dryers or vacuum dryers. Preferably, the drying step III) and wet granulation process are performed in one and the same apparatus. The granules are then dried and optionally sieved. Alternatively, the sieving is carried out before the drying.

In a further embodiment, the process of the present invention comprises a step IV) of adding to the (dried) granules from step III) or to the blended mixture or granules (obtained by dry granulation) from step II) a second disintegrant c) and optionally a pharmaceutical excipient d).

In a preferred embodiment, the components from step III) and/or from step II) and/or the second disintegrant c) and optionally the pharmaceutical excipient d) from step IV) can be sieved before being blended.

In a preferred embodiment, step IV) can be carried out by mixing the granules from step III) or from step II) with the second disintegrant c) and optionally the pharmaceutical excipient d), for example in free-fall mixers or compulsory mixers.

In an alternative embodiment, step IV) can be conducted such that the adding of the second disintegrant c) and optionally the pharmaceutical excipient d) to the granules from step III) or step II) can be conducted with only a part of the second disintegrant c) and optionally the pharmaceutical excipient d) in a mixing device, for example in a high shear or tumbler mixer. After this first mixing step, a next part of the second disintegrant c) and optionally the pharmaceutical excipient d) can be added, which may be followed by a next mixing step. This procedure can be repeated until the last part of the second disintegrant c) and optionally the pharmaceutical excipient d) is used, preferably one to five times.

In a further embodiment, step IV) can be carried out by mixing the blended mixture from step II) with the second disintegrant c) and optionally the pharmaceutical excipient d), for example in conventional mixing devices. Preferably, step IV) is carried as explained for the mixing of the components of step I) above.

In step V), the mixture from step IV) can be compressed to form a compressed dosage form, preferably a tablet.

The compression of the mixture from step IV) can preferably be a direct compression. This direct compression step can preferably be carried out on a rotary press.

If a rotary press is applied, the main pressure can range from 2 to 30 MPa, preferably from 3 to 25 MPa, more preferably from 5 to 15 MPa.

In optional step VI), the dosage form, preferably the tablet, can be film coated.

The tablet may be a tablet which can be swallowed unchewed. It may likewise be a chewable tablet or a dispersible tablet. In the case of tablets which are swallowed unchewed, it is preferable that they be coated with a film layer.

Typical film-coatings are preferably formed using polymers such as shellack, modified celluloses, polyvinylpyrrolidone, polymethacrylates and/or polyvinyl acetate phthalate.

The thickness of the coating is preferably 1 to 100 μm, more preferably 5 to 80 μm.

In a further embodiment of the invention, the process is characterized in that the obtained compressed solid dosage form comprises the pressure sensitive solid form of the active pharmaceutical ingredient as the only solid form of said active pharmaceutical ingredient.

Preferably, the active pharmaceutical ingredient in step I) is the same as after the compression step V). This means that an XRPD of the pressure sensitive solid form of the active pharmaceutical ingredient taken in step I) and an XRPD taken after step V) are identical within statistical error and/or if differences due to changes in sample orientation are appropriately considered.

Further, the invention relates to granules obtained by a process comprising the steps of

j) providing a pressure sensitive solid form of an active pharmaceutical ingredient a), a first disintegrant b) having elastic properties and optionally a further pharmaceutical excipient,
jj) mixing and granulating the mixture from step j) with a granulation liquid,
jjj) drying the granules from step jj),

wherein a pressure sensitive solid form is defined as a form which modifies its degree of structural order under a pressure of 30 MPa.

Step j) corresponds to step I) as described above.

In step jj), the mixture of step j) is mixed and granulated with a granulation liquid. The wet granulation step is described above in relation with step II). The drying step jjj) is described above in relation with optional step III).

The invention will now be explained with reference to the following examples.

**EXAMPLE**

Compressed solid dosage form comprising Form I of idelalisib

Compressed tablets of the following compositions are prepared:

| | 135 mg tablet | 220 mg tablet | 440 mg tablet |
|---|---|---|---|
| *Inner phase granules* | | | |
| Idelalisib Form I (API) | 100 mg | 150 mg | 300 mg |
| Microcrystalline Cellulose (Filler) | 9 mg | 18 mg | 36 mg |
| Croscarmellose Sodium (First Disintegrant) | 14 mg | 28 mg | 56 mg |
| Hydroxypropyl Cellulose (Binder) | 4 mg | 8 mg | 16 mg |
| *Outer phase* | | | |
| Colloidal silica (Glidant) | 3 mg | 6 mg | 12 mg |
| Sodium Starch Glycolate (Second Disintegrant) | 3 mg | 6 mg | 12 mg |
| Magnesium Stearate (Lubricant) | 2 mg | 4 mg | 8 mg |

In a first step, Form I of idelalisib, microcrystalline cellulose and croscarmellose sodium are mixed together for 15 min in a standard high shear mixer. Hydroxypropyl cellulose is dissolved in water. Granulation process is performed by adding granulation liquid to the dry mixture under stirring in a standard high shear mixer. The wet granules are sieved over 800 μm and are then dried in a fluidized bed drier at 40°C for 3 hours. Colloidal silica, Sodium starch glycolate and magnesium stearate are added to the mixture and the final blend is mixed for another 5 minutes. The mixture is compressed on a single punch press leading to tablets of the above-mentioned composition, respectively.

**Claims**

**1.** A compressed solid dosage form comprising:

a) a pressure sensitive solid form of an active pharmaceutical ingredient,
b) a first disintegrant having elastic properties,
c) a second disintegrant, and
d) optionally a pharmaceutical excipient,
f) optionally a coating,

wherein the weight ratio of the pressure sensitive solid form of an active pharmaceutical ingredient a) to the first disintegrant having elastic properties b) is from 10:1 to 1:7 and wherein a pressure sensitive solid form is defined as a form which modifies its degree of structural order under a pressure of 30 MPa.

**2.** A compressed solid dosage form according to claim 1, wherein the first disintegrant b) has a brittle fracture index of <0.15.

**3.** A compressed solid dosage form according to claim 1 or 2, wherein the active pharmaceutical ingredient a) is selected from the BCS class II or IV.

**4.** A compressed solid dosage form according to any one of claims 1 to 3, wherein the weight ratio of the pressure sensitive solid form of an active pharmaceutical ingredient a) to the first disintegrant having elastic properties b) is from 5:1 to 1:5.

**5.** A compressed solid dosage form according to any one of claims 1 to 4, wherein the pressure sensitive solid form of an active pharmaceutical ingredient a) and the first disintegrant having elastic properties b) are associated with one another in the form of granules.

6. A compressed solid dosage form according to any one of claims 1 to 5, wherein the pressure sensitive solid form of the active pharmaceutical ingredient a) is a polymorphic form which converts into another polymorphic form upon pressure.

7. A compressed solid dosage form according to any one of claims 1 to 6, wherein the pressure sensitive solid form of the active pharmaceutical ingredient a) is an amorphous form which converts into a crystalline form upon pressure.

8. A compressed solid dosage form according to any one of claims 1 to 7, wherein the active pharmaceutical ingredient a) is selected from caffeine, indomethacin, sulfobenzamid, paroxetine, theophylline, amoxicillin, cephalexin, cefixime, piroxicam, acetaminophen, chlorpropamid and maprotiline.

9. A compressed solid dosage form according to any one of claims 1 to 8, wherein the first disintegrant b) is selected from a cross-linked cellulose derivative, preferably croscarmellose sodium, or non cross-linked cellulose derivative.

10. A compressed solid dosage form according to any one of claims 1 to 9, wherein the second disintegrant c) is selected from starch, starch derivatives, cellulose, cellulose derivatives, polyvinylpyrrolidone, respectively either cross-linked or not cross-linked, and mixtures thereof.

11. A compressed solid dosage form according to any one of claims 1 to 10, wherein the pharmaceutical excipient d) is a filler, binder, lubricant, glidant, sweetener, flavour or a mixture thereof.

12. A compressed solid dosage form according to any one of claims 1 to 11, wherein the tablet releases 70% to 100% of the active pharmaceutical ingredient a) after 15 min, as measured according to USP app. II at 37°C, 50 rpm, 0.1M HCl, 900 mL.

13. A compressed solid dosage form according to any one of claims 1 to 12, comprising

a) 5 to 75 wt.% of pressure sensitive solid form of an active pharmaceutical ingredient,
b) 1 to 25 wt.% of the first disintegrant,
c) 1 to 15 wt.% of the second disintegrant,
d) 0 to 55 wt.% of a pharmaceutical excipient,

based on the total weight of the dosage form.

14. A compressed solid dosage form according to any one of claims 5 to 13, in as far as claims 6 to 13 refer back to claim 5, wherein the granules are obtainable by wet granulation of the pressure sensitive solid form of the active pharmaceutical ingredient a) with the first disintegrant b) and optionally the further pharmaceutical excipient d).

15. Process for the production of a compressed solid dosage form comprising a pressure sensitive solid form of an active pharmaceutical ingredient comprising the steps of

I) providing a pressure sensitive solid form of an active pharmaceutical ingredient a), a first disintegrant having elastic properties b) and optionally a further pharmaceutical excipient d),
II) mixing, and preferably granulating, the mixture of step I), preferably with a granulation liquid, and
III) optionally drying the granules from step II),

wherein a pressure sensitive solid form is defined as a form which modifies its degree of structural order under a pressure of 30 MPa.

16. Process for the production of a compressed solid dosage form according to claim 15, further comprising the step of

IV) adding to the granules of step III) a second disintegrant c) and optionally a further pharmaceutical excipient d).

17. Process for the production of a compressed solid dosage form according to claim 16, further comprising the steps

V) compressing the mixture of step IV) to form a compressed dosage form,
VI) optionally film coating the dosage form.

**18.** Process according to any one of claims 15 to 17, wherein the obtained compressed solid dosage form comprises the pressure sensitive solid form of the active pharmaceutical ingredient as the only solid form of said active pharmaceutical ingredient.

**19.** Granules obtainable by a process comprising the steps of

j) providing a pressure sensitive solid form of an active pharmaceutical ingredient a), a first disintegrant b) having elastic properties and optionally a further pharmaceutical excipient d),
jj) mixing and granulating the mixture from step j) with a granulation liquid,
jjj) drying the granules from step jj),

wherein a pressure sensitive solid form is defined as a form which modifies its degree of structural order under a pressure of 30 MPa.

**FIG. 1**

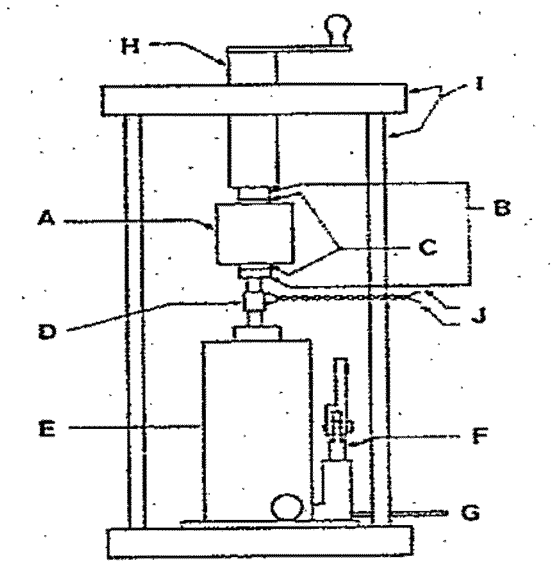


Schematic of apparatus for tensile strength determination by transverse compression.

Europäisches Patentamt
European Patent Office
Office européen des brevets

# EUROPEAN SEARCH REPORT

Application Number
EP 14 17 9182

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2008/311162 A1 (DARMUZEY OLIVIA [BE] ET AL) 18 December 2008 (2008-12-18)<br>* paragraph [0006] *<br>* claims 1-36 *<br>----- | 1-19 | INV.<br>A61K9/20<br>A61K31/52 |
| Y | US 2010/062062 A1 (MCMILLAN BRIAN ROBERT [US] ET AL) 11 March 2010 (2010-03-11)<br>* paragraph [0011] - paragraph [0015] *<br>* paragraph [0009] *<br>* claims 1-22 *<br>----- | 1-19 | |
| | | | TECHNICAL FIELDS SEARCHED (IPC) |
| | | | A61K |

The present search report has been drawn up for all claims

2

| Place of search | Date of completion of the search | Examiner |
|---|---|---|
| Munich | 25 September 2014 | Sindel, Ulrike |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 14 17 9182

This annex lists the patent family members relating to the patent documents cited in the above-mentioned European search report. The members are as contained in the European Patent Office EDP file on
The European Patent Office is in no way liable for these particulars which are merely given for the purpose of information.

25-09-2014

| Patent document cited in search report | Publication date | Patent family member(s) | Publication date |
|---|---|---|---|
| US 2008311162 A1 | 18-12-2008 | NONE | |
| US 2010062062 A1 | 11-03-2010 | US 2010062062 A1 | 11-03-2010 |
| | | WO 2010030735 A2 | 18-03-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013134288 A


### Non-patent literature cited in the description


- **GHAN G. A. ; LALLA J. K.** Effect of compressional forces on piroxicam polymorphs. *J. Pharm. Pharmacol.,* 1992, vol. 44, 678-681
- **D. L. SIMMONS ; R. J. RANZ ; N. D. GYANCHANDANI.** *Can. J. Pharm. Sci.,* 1973, vol. 8, 125
- **E. HIESTAND ; C. PEOT.** Tensile strength of compressed powders and an example of incompatibility as endpoint on shear yield locus. *J Pharm Sci,* 1974, vol. 63, 605-612